# EUROPÄISCHE PATENTSCHRIFT

(11) **EP 3 829 482 B1**
(45) Veröffentlichungstag und Bekanntmachung des Hinweises auf die Patenterteilung: **06.11.2024**
(21) Anmeldenummer: 19749339.8
(22) Anmeldetag: 02.08.2019
(51) Int. Cl.: A61C 13/00, A61C 13/09, C03C 3/076, C03C 3/097, C03C 4/00, C03C 10/00, C04B 41/00

(54) **MEHRFARBIGER ROHLING FÜR DENTALE ZWECKE**
MULICOLORED BLANK FOR DENTAL PURPOSES
ÉBAUCHE MULTICOLORE À DES FINS DENTAIRES

(30) Priorität: 02.08.2018 EP 18187024
(43) Veröffentlichungstag der Anmeldung: 09.06.2021
(73) Patentinhaber: Ivoclar Vivadent AG, 9494 Schaan (LI)
(72) Erfinder: SEGER, Jürgen, 9490 Vaduz (LI); BÜRKE, Harald, 6820 Frastanz (AT); KROLIKOWSKI, Sebastian, 8853 Lachen (CH); ROTHBRUST, Frank, 6822 Röns (AT)
(74) Vertreter: Uexküll & Stolberg
(86) Internationale Anmeldenummer: PCT/EP2019/070891
(87) Internationale Veröffentlichungsnummer: WO 2020/025795

(56) Entgegenhaltungen:
- EP-A1- 2 065 012
- EP-A1- 3 053 541
- EP-A1- 3 064 170
- EP-B1- 2 065 012
- WO-A1-02/09612
- WO-A1-2015/051095
- WO-A1-2015/173394
- WO-A1-2017/182173
- WO-A1-2018/073273
- CH-A1- 711 813
- DE-A1- 102015 122 865
- DE-A1- 102016 119 934
- JP-B2- 5 662 914
- US-A1- 2002 022 563
- US-A1- 2003 073 563
- US-A1- 2007 042 889
- US-A1- 2013 069 264
- US-A1- 2013 221 554
- US-A1- 2013 224 454
- US-A1- 2015 024 345
- US-A1- 2015 104 655
- US-A1- 2015 140 274
- US-A1- 2015 282 905
- US-A1- 2016 236 971
- US-A1- 2016 262 860
- US-A1- 2017 056 140
- US-A1- 2017 088 456
- US-A1- 2017 273 764
- US-A1- 2017 340 420

## Beschreibung

Die Erfindung betrifft einen Rohling für dentale Zwecke, mit dem die optischen Eigenschaften von natürlichem Zahnmaterial sehr gut nachgeahmt werden können und der sich aufgrund seiner Eigenschaften insbesondere für die einfache Herstellung von ästhetisch anspruchsvollen Dentalrestaurationen mit sehr guten mechanischen Eigenschaften eignet.

Es stellt eine große Herausforderung dar, Rohlinge zu schaffen, die den vielfältigen Anforderungen für einen Einsatz im Bereich der Dentaltechnik gerecht werden. Derartige Rohlinge sollen nicht nur einfach herstellbar sein, sondern sie sollen auch einfach zur gewünschten Geometrie formbar sein und trotzdem hochfeste Restaurationen ergeben. Schließlich sollen bereits die Rohlinge eine Optik aufweisen, die der des natürlichen Zahnmaterials nahekommt, damit eine aufwendige nachträglich Verblendung der Restauration entfallen kann.

Mehrfarbige Blöcke, die den Verlauf der Farbe und der Transluzenz vom Dentin zum Zahnschmelz simulieren, und deren Einsatz in der Dentaltechnik sind aus dem Stand der Technik bekannt. Beispielsweise beschreibt die EP 0 870 479 A2 ein Verfahren zur Herstellung eines mehrfarbigen Formkörpers für die Weiterverarbeitung zu einer Zahnrestauration, bei dem mindestens zwei unterschiedlich gefärbte, pulver- oder granulatförmige Ausgangsmaterialien in eine Pressmatrize gefüllt und zu einem mehrfarbigen Formkörper gepresst werden.

Bei derartigen Formkörpern, die wegen ihrer üblichen Weiterverarbeitung durch Sintern auch als Sinterblöcke bezeichnet werden, kann ein Farbverlauf vergleichsweise einfach erreicht werden, indem die unterschiedlich gefärbten Ausgangsmaterialien nacheinander in die Pressmatrize gefüllt werden. Um den Farb- und Transluzenzverlauf eines natürlichen Zahnes möglichst genau zu imitieren, ist es jedoch meist erforderlich, mehr als zwei Schichten übereinander anzuordnen. Des Weiteren können bei uniaxial gepressten Blöcken zwei oder mehrere Schichten üblicherweise nur im Wesentlichen horizontal übereinander gepresst werden. Dabei besteht der weitere Nachteil, dass bei diskreten Schichten deren Übergang in der endgültigen Restauration noch sichtbar bleibt. Damit der natürliche Farb- und Transluzenzverlauf reproduziert werden kann, ist für den Ersatz eines Seitenzahnes gewöhnlich das Übereinanderschichten von mindestens drei Schichten erforderlich. Grundsätzlich gilt, dass der Übergang zwischen den Schichten umso weniger erkennbar ist, je mehr Schichten aufeinander geschichtet werden. Um den Übergang zwischen den einzelnen Schichten komplett zu eliminieren, ist es bei Sinterblöcken daher in der Regel erforderlich, zumindest den Übergang von Dentin zur Schneide kontinuierlich zu gestalten. Hierzu bedarf es jedoch üblicherweise aufwendiger Füllvorrichtungen, wie sie beispielsweise in der WO 2013/067994 A1 beschrieben sind.

Mit dem oben beschriebenen Verfahren zur Herstellung von uniaxial gepressten Sinterblöcken können Seitenzähne recht gut imitiert werden. Die Reproduktion eines Frontzahnes gestaltet sich in der Regel jedoch schwieriger, da horizontal geschichtete Blöcke nicht wie ein natürlicher Frontzahn aufgebaut sind, bei dem die transluzente Schmelzschicht im Wesentlichen über den gesamten Verlauf des Zahnes in zervikaler-inzisaler Richtung über dem farbintensiveren Dentinkern liegt.

Neben einem im Wesentlichen horizontalen Verlauf der verschiedenfarbigen Schichten sind daher auch Blöcke bekannt, bei denen die Grenzfläche zwischen den unterschiedlich eingefärbten Schichten eine parabelförmige Gestalt annimmt. Beispielsweise beschreiben die US 2013224454 und die WO 2015/051095 A1 einen derartigen Rohling und dessen Verwendung zur Herstellung von Zahnersatz.

Allerdings ist bei den bekannten mehrfarbigen Blöcken aus Pulver- oder Granulatschichten, wie z.B. Blöcken aus Zirkonoxid, unabhängig von dem Verlauf der Grenzschicht zwischen einzelnen Schichten nach der Formgebung zur Dentalrestauration noch ein Sinterschritt erforderlich, um die für eine Dentalrestauration zwingend erforderliche hohe Festigkeit zu erzielen. Diese Sinterung ist jedoch mit einer deutlichen Schrumpfung verbunden, die durch den Einsatz von vergrößerten Formen aufwendig berücksichtigt werden muss. Als Folge des Sinterprozesses können somit Passungsprobleme auftreten.

Um potentielle Passungsprobleme zu vermeiden sind neben Sinterblöcken grundsätzlich auch Glas- und Glaskeramikblöcke und deren Verwendung zur Herstellung von Zahnersatz bekannt, die mittels Massivglastechnologie erzeugt werden, d.h. durch Schmelzen von geeigneten Ausgangskomponenten, Gießen der Schmelze in eine geeignete Form und ggf. anschließende Wärmebehandlung. Bei diesen Blöcken kann auf einen Sinterschritt nach der Formgebung verzichtet werden. Allerdings sind derartige Glaskeramikblöcke, wie z.B. Blöcke aus Lithiumsilikatglaskeramik, üblicherweise monochrom, so dass bei deren Verwendung im Frontzahnbereich vom Zahntechniker ein Schichtmaterial aufgetragen werden muss, um den Farbverlauf eines natürlichen Zahnes möglichst gut nachzuahmen. Das Aufbringen eines Schichtmaterials ist ein aufwendiges und kostenintensives Verfahren. Zudem sind die mechanischen Eigenschaften des Schichtmaterials, wie z.B. dessen Festigkeit, in der Regel geringer als die des Gerüstmaterials, so dass das Schichtmaterial häufig die Ursache für das klinische Versagen einer Dentalrestauration darstellt. Ein einheitliches, monolithisches, hochfestes Glaskeramikmaterial, bei dem sich Dentinbereiche und Schneidebereiche ausschließlich in der Farbe und Transluzenz unterscheiden, aber ansonsten aus identischem Material bestehen, ist daher grundsätzlich wünschenswert.

Massivglasblöcke mit verschieden eingefärbten Glasschichten können zwar auf unterschiedliche Art und Weise hergestellt werden. Beispielsweise beschreibt die WO 2014/124879 A1 einen Rohling für dentale Zwecke, der zwei oder mehrere unterschiedlich gefärbte Schichten von Lithiumsilikatglas oder Lithiumsilikatglaskeramik aufweist und dadurch erhalten werden kann, dass eine monolithische Schicht in Form einer Schmelze auf eine andere monolithische Schicht mit unterschiedlicher Farbe aufgebracht wird. Um bei Massivglasblöcken einen scheinbar kontinuierlichen Farb- und Transluzenzverlauf zu erzielen, ist es jedoch in der Regel notwendig, sehr viele Schichten, wie z.B. acht Schichten oder mehr, aneinander zu fügen.

Bei den bekannten mehrfarbigen Rohlingen ist somit die Verwendung von einer Vielzahl von verschiedenfarbigen Schichten oder sogar kontinuierlichen Farbverläufen erforderlich, um den Farb- und Transluzenzverlauf eines natürlichen Zahnes möglichst realitätsgetreu nachzuahmen und eine sichtbare Grenzfläche zwischen einzelnen Schichten zu vermeiden. Die Bereitstellung von einer Vielzahl von verschiedenfarbigen Schichten ist jedoch insbesondere bei Blöcken, die über Massivglastechnologie hergestellt worden sind, aufwendig und selten wirtschaftlich. Bei mehrfarbigen Rohlingen aus Pulver- oder Granulatschichten, sog. Sinterblöcken, kommt als weiteres Problem hinzu, dass die erforderliche Sinterung nach der Formgebung der Dentalrestauration zu Passungsproblemen führen kann. Auch weisen die aus Sinterblöcken hergestellten Dentalrestaurationen grundsätzlich eine geringere Festigkeit auf.

Erfindungsgemäß sollen die oben genannten Probleme vermieden werden. Der Erfindung liegt insbesondere die Aufgabe zugrunde, einen Rohling zur Verfügung zu stellen, der einfach herstellbar ist, mit dem die optische Erscheinung von natürlichem Zahnmaterial sehr gut nachgeahmt werden kann, dem maschinell in einfacher Weise die Form der gewünschten Dentalrestauration gegeben werden kann und der nach der Formgebung ohne wesentliche Schrumpfung zu einer präzisen und hochfesten Dentalrestauration umgewandelt werden kann, bei der keine Grenzfläche zwischen verschiedenen Schichten sichtbar ist.

Diese Aufgabe wird durch den Rohling nach den Ansprüchen 1 bis 18 gelöst. Gegenstand der Erfindung ist ebenfalls das Verfahren zur Herstellung des Rohlings nach den Ansprüchen 19 bis 21, das Verfahren zur Herstellung von Dentalrestaurationen nach den Ansprüchen 22 bis 26 sowie die Verwendung des Rohlings nach Anspruch 27.

Der erfindungsgemäße Rohling für dentale Zwecke zeichnet sich dadurch aus, dass er eine erste und eine zweite Schicht aufweist, die unabhängig voneinander auf Basis von
Glas,
Glaskeramik oder
Keramik
sind, wobei die erste Schicht und die zweite Schicht sich in der Farbe unterscheiden und eine Grenzfläche bilden, wobei die Grenzfläche schräg verläuft.

Der Begriff "auf Basis von" bedeutet im obigen Zusammenhang, dass die erste und die zweite Schicht des Rohlings, bezogen auf die Masse der Summe aller Bestandteile der Schicht, überwiegend Glas, Glaskeramik oder Keramik enthalten. Es ist bevorzugt, dass die erste und die zweite Schicht unabhängig voneinander aus Glas, Glaskeramik oder Keramik bestehen.

Vorzugsweise sind sowohl die erste als auch die zweite Schicht beide auf Basis von Glas, beide auf Basis von Glaskeramik oder beide auf Basis von Keramik. Weiterhin ist es bevorzugt, dass sowohl die erste und als auch die zweite Schicht beide aus Glas, beide aus Glaskeramik oder beide aus Keramik bestehen.

Mit Unterschieden in der Farbe sind Unterschiede im Farbton im engeren Sinne und/oder Unterschiede in der Transluzenz, Opaleszenz oder Fluoreszenz gemeint. Der Begriff "Transluzenz" beschreibt dabei die Lichtdurchlässigkeit. Die Farbe kann insbesondere durch ihren Lab-Wert oder durch einen in der Dentalindustrie üblichen Farbschlüssel charakterisiert werden. Des Weiteren ist es nicht notwendig, dass die Unterschiede in der Farbe der ersten und zweiten Schicht im Rohling mit dem menschlichen Auge zu erkennen sind. Vielmehr kann ein Unterschied im Farbton und/oder der Transluzenz erst nach einem Sinterschritt oder einer Wärmebehandlung sichtbar werden.

Der erfindungsgemäße Rohling zeichnet sich somit insbesondere dadurch aus, dass bereits mit lediglich zwei verschiedenfarbigen Schichten ein gewünschter Farbverlauf erzeugt wird, so dass der Farb- und Transluzenzverlauf von natürlichen Zähnen, insbesondere Frontzähnen, nachgeahmt werden kann, ohne dass im späteren Zahnersatz die Grenzfläche zwischen den Schichten sichtbar bleibt. Dies wird überraschenderweise dadurch erreicht, dass die Grenzfläche zwischen der ersten und zweiten Schicht anders als bei uniaxial verpressten Sinterblöcken nicht horizontal, sondern schräg verläuft. Dadurch ist es möglich, eine transluzentere Schicht, die die Schneideschicht imitieren soll, schräg oberhalb einer opakeren und stärker gefärbten Schicht, die die Dentinschicht imitieren soll, anzuordnen, so dass der Farbverlauf eines natürlichen Zahns, insbesondere Frontzahnes, nachgeahmt werden kann und der Übergang kontinuierlich erscheint, ohne dass eine störende Grenzlinie zwischen den Schichten erkennbar wird.

Das Vorhandensein weiterer Schichten ist erfindungsgemäß nicht ausgeschlossen. Allerdings ist der präparative Aufwand zur Herstellung der Rohlinge geringer, je weniger Schichten der Rohling umfasst. Somit ist es bevorzugt, dass der erfindungsgemäße Rohling neben der ersten und der zweiten Schicht keine weiteren Schichten aufweist.

Vorzugsweise sind die durch die erste und zweite Schicht gebildeten Volumenbereiche nicht konzentrisch. Das heißt, dass diese Volumenbereiche des Rohlings nicht denselben Schwerpunkt besitzen.

Es ist weiterhin bevorzugt, dass in einer ersten Schnittebene durch den Rohling, die parallel zur Einschubachse des Rohlings verläuft, die Grenzfläche zwischen der ersten und zweiten Schicht nicht senkrecht zur Einschubachse verläuft.

Der Begriff "Einschubachse des Rohlings" bezeichnet die Achse in zervikal-inzisaler Richtung und beschreibt insbesondere für den Fall, dass aus dem Rohling eine Krone herzustellen ist, die Einführungsrichtung des aus dem Rohling herzustellenden Zahnersatzes zu einem Modell. Im Allgemeinen fällt die Einschubachse des Rohlings somit im Wesentlichen mit der Zahnlängsachse des daraus herzustellenden Zahnersatzes überein. Mithin ist die Einschubachse auf die jeweilige Patientensituation bezogen. Im Falle eines Rohlings in Form eines Blockes oder Quaders stellt die Einschubachse vorzugsweise die Gerade dar, die durch die Schwerpunkte von zwei gegenüberliegenden Seitenflächen des Rohlings verläuft, insbesondere von zwei in zervikal-inzisaler Richtung gegenüberliegenden Seitenflächen. Im Falle eines Rohlings in Form einer Scheibe oder eines Zylinders verläuft die Einschubachse vorzugsweise senkrecht zur Scheibenfläche. Weist der Rohling einen Halter für eine Bearbeitungsvorrichtung auf, wie z.B. einen Halter für eine CAD/CAM-Vorrichtung, so verläuft die Einschubachse des Rohlings - wie weiter unten beschrieben - gemäß einer Ausführungsform vorzugsweise senkrecht zu der Rotationsachse des Halters.

Insbesondere ist es bevorzugt, dass die Grenzfläche in der ersten Schnittebene im Wesentlichen geradlinig verläuft. Mithin ist der erfindungsgemäße Rohling in einer bevorzugten Ausführungsform dadurch gekennzeichnet, dass die Grenzfläche der ersten und zweiten Schicht in der ersten Schnittebene im Wesentlichen geradlinig verläuft und einen von 90° verschiedenen Winkel zur Einschubachse aufweist.

Besonders bevorzugt ist ein Rohling, bei dem die Grenzfläche der ersten und zweiten Schicht in der ersten Schnittebene einen Winkel von 20 bis 80°, vorzugsweise 30 bis 80°, zur Einschubachse aufweist. Bei einem derartigen Verlauf der Grenzfläche kann der natürliche Farbverlauf eines Frontzahnes besonders gut nachgeahmt werden.

In einer anderen Ausführungsform verläuft die Grenzfläche der ersten und zweiten Schicht in der ersten Schnittebene bogenförmig. Auch in dieser Ausführungsform ist es bevorzugt, dass die Grenzfläche der ersten und zweiten Schicht in der ersten Schnittebene einen von 90° verschiedenen Winkel zur Einschubachse des Rohlings aufweist. Besonders bevorzugt weist die Ausgleichsgerade durch die Linie der Grenzfläche in der ersten Schnittebene einen Winkel von 20 bis 80°, vorzugsweise 30 bis 80°, zur Einschubachse des Rohlings auf, um den Farbverlauf eines Frontzahnes besonders gut nachahmen zu können.

Unabhängig davon, ob die Grenzfläche der ersten und zweiten Schicht in der ersten Schnittebene im Wesentlichen geradlinig oder bogenförmig verläuft, weist der Rohling einen ersten und zweiten Bereich auf, wobei sich der erste Bereich auf der einen Seite der Grenzfläche, z.B. oberhalb der Grenzfläche, befindet und dazu dient, in der aus dem Rohling herzustellenden Dentalrestauration die Schneide eines Zahnes zu imitieren, und sich der zweite Bereich auf der gegenüberliegenden Seite der Grenzfläche, z.B. unterhalb der Grenzfläche, befindet und dazu dient, in der aus dem Rohling herzustellenden Dentalrestauration das Dentin eines Zahnes zu imitieren.

Des Weiteren ist es bevorzugt, dass die Rotationsachse des Rohlings im Wesentlichen senkrecht zur Einschubachse des Rohlings verläuft. Besonders bevorzugt beträgt der Winkel zwischen der Rotationsachse und der Einschubachse in der ersten Schnittebene 90°. In einer weiteren Ausführungsform beträgt der Winkel zwischen der Rotationsachse und der Einschubachse in der ersten Schnittebene vorzugsweise 70 bis 110°, insbesondere 80 bis 100° und besonders bevorzugt etwa 90°. Der Begriff "Rotationsachse des Rohlings" bezeichnet die Achse, um die der Rohling bei der maschinellen Bearbeitung zur Formgebung der gewünschten Dentalrestauration gedreht wird. Weist der Rohling z.B. einen Halter für eine CAD/CAM-Vorrichtung auf, so fällt die Rotationsachse des Rohlings mit der Rotationsachse des Halters überein.

Mithin ist ein Rohling bevorzugt, bei dem die Grenzfläche der ersten und der zweiten Schicht in der ersten Schnittebene einen Winkel von 10 bis 70°, vorzugsweise 10 bis 60°, zur Rotationsachse aufweist. Es wurde gefunden, dass ein derartiger Winkel zwischen der Grenzfläche der ersten und zweiten Schicht und der Rotationsachse zu mehr Freiheitsgraden bei der Gestaltung der Zahnrestauration und deren Platzierung im Rohling führt, so dass auf eine aufwendige 5-achsige maschinelle Bearbeitung des Rohlings verzichtet werden kann.

Ferner ist es bevorzugt, dass die Grenzfläche der ersten und zweiten Schicht in einer zweiten Schnittebene durch den Rohling, die senkrecht zur ersten Schnittebene verläuft, bogenförmig verläuft und insbesondere konvex gewölbt verläuft. Dabei bezieht sich der Begriff "konvex gewölbt" auf die Schicht, die eine geringere Transluzenz aufweist und somit die Dentinschicht des zu ersetzenden Zahnes imitieren soll. D.h. die weniger transluzente Schicht ist in der zweiten Schnittebene nach "außen", d.h. in Richtung der Schicht mit höherer Transluzenz gewölbt. Auch dieser Verlauf der Grenzfläche zwischen erster und zweiter Schicht führt dazu, dass der natürliche Farbverlauf eines Frontzahnes besonders gut nachgeahmt werden kann. Insbesondere führt die konvexe Krümmung in Bezug auf die Dentinschicht dazu, dass bei der Reproduktion einer Frontzahnkrone nicht nur inzisal, sondern auch mesial und distal ein kontinuierlich erscheinender Übergang zwischen Dentin und Schneide ermöglicht wird.

Bei natürlichen Frontzähnen läuft das Dentin im inzisalen Bereich in einer Mamelonstruktur aus. Um Frontzähne besonders realitätsgetreu nachzuahmen, ist es daher bevorzugt, dass bei dem erfindungsgemäßen Rohling die Grenzfläche der ersten und zweiten Schicht in der zweiten Schnittebene eine Mamelonstruktur aufweist. Der Begriff "Mamelon" bezeichnet dabei Höckerchen oder Hügel. Mithin weist die Mamelonstruktur Einkerbungen auf. Insbesondere ist es vorteilhaft, dass die Mamelons in zervikaler Richtung auslaufen. Des Weiteren ist es vorteilhaft, dass die Mamelons in inzisaler Richtung spitz zulaufen. Die Grenzfläche weist gemäß einer bevorzugten Ausführungsform in der zweiten Schnittebene somit ausgehend von ihrem grundsätzlich konvex gewölbten Verlauf vorzugsweise eine Vielzahl von Einkerbungen auf. Vorzugsweise unterscheiden sich die Einkerbungen in ihrer Breite und Tiefe voneinander, so dass vorzugsweise eine unregelmäßige Mamelonstruktur vorliegt. Weiterhin ist die Mamelonstruktur vorzugsweise derartig gestaltet, dass die Tiefe der Einkerbungen bis zu 2 mm, vorzugsweise 0,1 bis 0,5 mm, beträgt. In einer Ausführungsform ist die Tiefe einer oder aller Einkerbungen der Mamelonstruktur nicht konstant, sondern verringert sich entlang einer zu der zweiten Schnittebene senkrecht verlaufenden Erstreckung der Grenzfläche. Wird der erfindungsgemäße Rohling zur Herstellung einer Krone zur Restaurierung eines Frontzahnes verwendet, weist die Grenzfläche vorzugsweise zwei oder drei Mamelons auf.

Des Weiteren ist ein Rohling bevorzugt, bei dem die erste Schicht einen Brechungsindex aufweist, der sich um nicht mehr als 0,1 von dem Brechungsindex der zweiten Schicht unterscheidet. Diese Ausführungsform hat den besonderen Vorteil, dass optisch keine störende Grenzfläche zwischen den Schichten wahrnehmbar ist. Der Brechungsindex kann über die Immersionsmethode mit Brechungsindexflüssigkeiten oder über ein sogenanntes Abbe-Refraktometer bestimmt werden. Im Fall von Rohlingen auf Basis von Lithiumsilikat-Glaskeramik bezieht sich die obige bevorzugte Differenz der Brechungsindices der ersten und zweiten Schicht auf den Lithiumdisilikatzustand, d.h. nach der Wärmebehandlung zur Ausbildung von Lithiumdisilikat unterscheidet sich der Brechungsindex der ersten Schicht um nicht mehr als 0,1 von dem Brechungsindex der zweiten Lithiumdisilikatschicht. Wie einem Fachmann bekannt ist, hängt der Brechungsindex eines Glases oder einer Glaskeramik von deren chemischer Zusammensetzung und/oder den ggf. vorhandenen Kristallphasen ab. Beispielsweise führt eine Erhöhung des Anteils von SiO₂ bei Gläsern auf Basis von SiO₂/CaO/MgO/Na₂O/Al₂O₃/K₂O zu einer Erniedrigung des Brechungsindex.

Außerdem ist es bevorzugt, dass der erfindungsgemäße Rohling eine Markierung aufweist, die von einer CAD/CAM-Vorrichtung erkannt wird und mit der die Lage der Grenzfläche der ersten und zweiten Schicht mit einer Genauigkeit von insbesondere 0,1 mm bestimmt werden kann. Die Gestaltung einer derartigen Markierung kann frei gewählt werden. Beispielsweise kann die Markierung in Form einer Kerbe oder Ausbuchtung an einer Kante oder auf einer Seitenfläche des Rohlings angebracht sein. Gemäß einer Ausführungsform stellt die Markierung eine Kerbe dar, die direkt an der Grenzfläche der ersten und der zweiten Schicht angebracht ist. Alternativ kann die Kerbe auch beabstandet von der Grenzfläche angeordnet sein. Wichtig ist in diesem Zusammenhang die Reproduzierbarkeit des Abstandes der Kerbe zur Grenzfläche, damit z.B. die CAD/CAM-Einheit die Restauration im richtigen Abstand zur Grenzfläche platziert, um den gewünschten Anteil an Dentin- und Schneideschicht zu erreichen. Markierungen in Form einer Kerbe können bei der maschinellen Bearbeitung des Rohlings durch ein Tastverfahren erkannt werden. Im Fall einer Markierung in Form eines Aufdrucks oder einer farblichen Markierung kann die Markierung durch eine Kamera oder einen Scanner erfasst werden. Die Beschreibung der Lage der Grenzfläche zwischen der ersten und der zweiten Schicht im Verhältnis zur Markierung kann auch in einem zu scannenden QR-Code oder Datenmatrix-Code integriert werden. Die CAD/CAM-Einheit kann die optimale Lage der Restauration im Rohling berechnen. Alternativ kann auch der Anwender mit Hilfe der CAD-Software die Lage der Restauration in den gewünschten Bereich des Rohlings manuell verschieben, insbesondere wenn der Schichtverlauf in der CAD-Software im Rohling dargestellt wird.

In einer besonders bevorzugten Ausführungsform weist der erfindungsgemäße Rohling eine Kombination von zwei oder mehreren der oben genannten, bevorzugten Merkmale auf. Insbesondere zeichnet sich der Rohling dadurch aus, dass
- die Grenzfläche der ersten und zweiten Schicht in der ersten Schnittebene im Wesentlichen geradlinig oder bogenförmig verläuft und einen von 90° verschiedenen Winkel zur Einschubachse aufweist, wobei der Winkel der Grenzfläche bzw. der Ausgleichsgerade durch den Verlauf der Grenzfläche in der ersten Schnittebene zur Einschubachse in der ersten Schnittebene 20 bis 80°, vorzugsweise 30 bis 80°, beträgt;
- die Rotationsachse des Rohlings in der ersten Schnittebene im Wesentlichen senkrecht zur Einschubachse verläuft, wobei der Winkel zwischen der Rotationsachse und der Einschubachse in der ersten Schnittebene vorzugsweise 70 bis 110°, insbesondere 80 bis 100° und besonders bevorzugt 90° beträgt;
- die Grenzfläche der ersten und zweiten Schicht in der ersten Schnittebene im Wesentlichen geradlinig oder bogenförmig verläuft und einen von 90° verschiedenen Winkel zur Rotationsachse aufweist, wobei der Winkel der Grenzfläche bzw. der Ausgleichsgerade durch den Verlauf der Grenzfläche in der ersten Schnittebene zur Rotationsachse in der ersten Schnittebene 10 bis 70°, vorzugsweise 10 bis 60°, beträgt;
- die Grenzfläche der ersten und zweiten Schicht in einer zweiten Schnittebene durch den Rohling, die senkrecht zur ersten Schnittebene verläuft, bogenförmig verläuft und insbesondere konvex gewölbt verläuft;
- die Grenzfläche der ersten und zweiten Schicht in der zweiten Schnittebene eine Mamelonstruktur aufweist, wobei die Mamelonstruktur vorzugsweise derartig gestaltet ist, dass die Tiefe der Einkerbungen der Mamelonstruktur zumindest in einem Teil der Grenzfläche bis zu 2 mm, vorzugsweise 0,1 bis 0,5 mm, beträgt;
- die erste Schicht einen Brechungsindex aufweist, der sich um nicht mehr als 0,1 von dem Brechungsindex der zweiten Schicht unterscheidet; und
- der erfindungsgemäße Rohling eine Markierung aufweist, die von einer CAD/CAM-Vorrichtung erkannt wird und mit der die Lage der Grenzfläche der ersten und zweiten Schicht mit einer Genauigkeit von insbesondere 0,1 mm bestimmt werden kann.

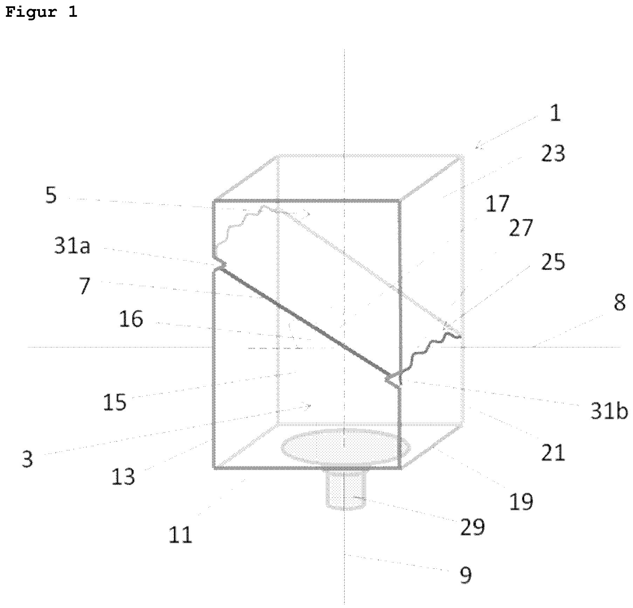

Beispielsweise zeigt Figur 1 eine schematische Ansicht eines mehrfarbigen Rohlings 1 gemäß der Erfindung, der eine erste Schicht 3 und eine zweite Schicht 5 aufweist, die sich in der Farbe unterscheiden und eine Grenzfläche 7 bilden. Die Grenzfläche 7 verläuft in einer ersten Schnittebene, die parallel zur Einschubachse 8 verläuft und in Figur 1 als durch Kanten 11 und 13 gebildete Seitenfläche 15 des Rohlings dargestellt ist, in einem Winkel 16 zur Einschubachse 8. Die Einschubachse 8 verläuft in der ersten Schnittebene 15 senkrecht zur Rotationsachse 9 des Rohlings. Dabei verläuft die Grenzfläche 7 in der ersten Schnittebene 15 in einem Winkel 17 zur Rotationsachse 9. In einer zweiten Schnittebene, die in Figur 1 durch die durch Kanten 19 und 21 gebildete Seitenfläche 23 dargestellt ist, verläuft die Grenzfläche 7 konvex gewölbt und weist eine Mamelonstruktur 25 mit Einkerbungen 27 auf. Der Rohling 1 weist ferner einen Halter 29 für die Befestigung in einer CAD/CAM-Vorrichtung sowie Markierungen 31a und 31b auf, welche von einer CAD/CAM-Vorrichtung erkannt werden können, so dass der Verlauf der Grenzfläche 7 von der CAD/CAM-Vorrichtung erkannt werden kann.

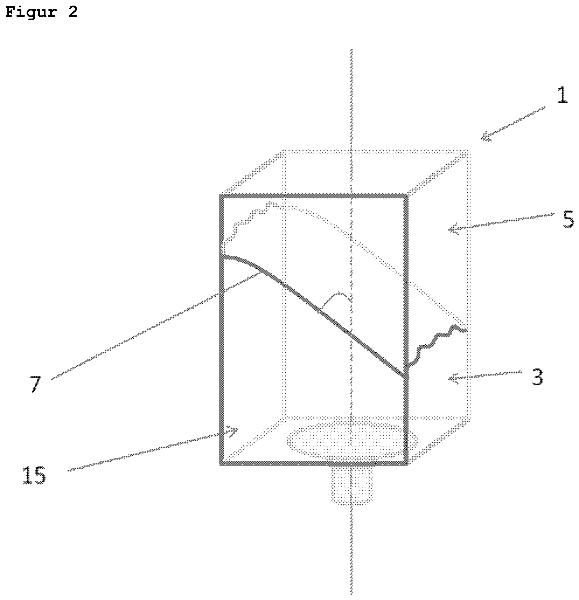

Figur 2 zeigt eine schematische Ansicht gemäß einer weiteren Ausführungsform eines mehrfarbigen Rohlings 1, in der die Grenzfläche 7 zwischen der ersten Schicht 3 und der zweiten Schicht 5 in der Schnittebene 15 nicht vollständig geradlinig, sondern zumindest teilweise bogenförmig verläuft.

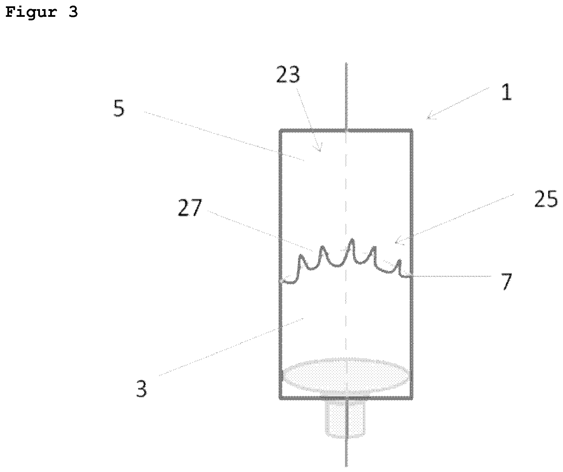

Figur 3 zeigt einen Querschnitt durch den mehrfarbigen Rohling 1 gemäß Figur 1 in der zweiten Schnittebene 23. Zu erkennen ist der grundsätzlich konvex gewölbte Verlauf der Grenzfläche 7 zwischen der ersten Schicht 3 und der zweiten Schicht 5. Dieser bogenförmige Verlauf wird durch die Mamelonstruktur 25 mit einzelnen Einkerbungen 27 überlagert.

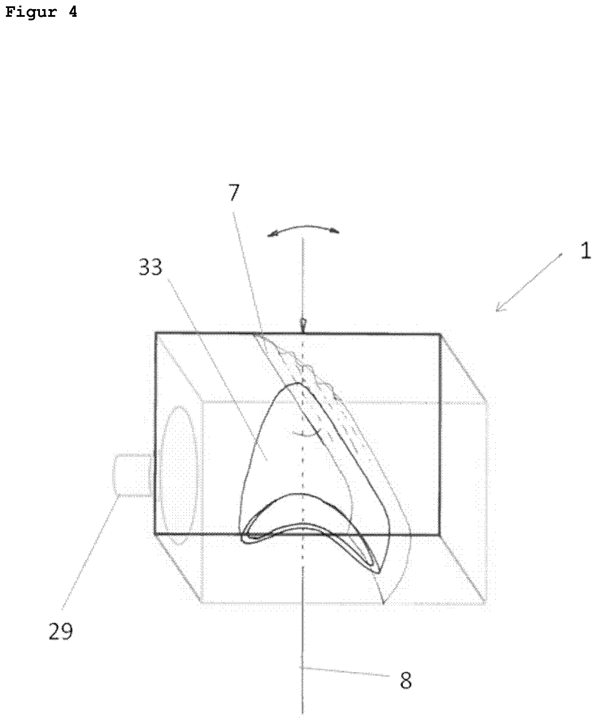

Figur 4 zeigt eine schematische Ansicht einer weiteren Ausführungsform des erfindungsgemäßen Rohlings 1, bei der die Einschubachse 8 senkrecht zu der Rotationsachse des Halters 29 verläuft. Außerdem ist die Lage der aus dem Rohling 1 herzustellenden Dentalrestauration 33 in dem Rohling 1 dargestellt, wobei ein Teil der Restauration 33, nämlich der Teil, der die Schneide in einem Frontbereich der Restauration 33 imitieren soll, oberhalb der Grenzfläche 7 liegt, und ein anderer Teil der Restauration 33, nämlich der Teil, der hauptsächlich die Dentinschicht imitieren soll, unterhalb der Grenzfläche 7 liegt.

In einer weiteren Ausführungsform kann die Grenzfläche mit Farbeffekten ausgestaltet sein. Dadurch kann ein nachträgliches Bemalen der aus dem Rohling herzustellenden Dentalrestauration vermieden werden. Der Farbeffekt kann beispielsweise einen Schmelzriss, einen Schmelzflecken oder eine andere Charakterisierung imitieren. Derartige Farbeeffekte können bei der Herstellung des Rohlings verwirklicht werden, indem sie auf die erste Schicht aufgebracht werden, bevor die zweite Schicht auf die erste Schicht aufgebracht wird. Auch 3D-Pulverdruckverfahren sind zur Verwirklichung der Farbeffekte geeignet, wobei der Effekt auf den Rohling gedruckt wird und dieser anschließend gesintert wird.

In Bezug auf das Material des erfindungsgemäßen Rohlings ist es bevorzugt, dass das Glas, die Glaskeramik oder die Keramik ausgewählt sind aus Lithiumsilikat-Glas, Lithiumsilikat-Glaskermik, Siliciumdioxid-Glas, Siliciumdioxid-Glaskeramik und/oder Zirkonoxid.

Gemäß einem ersten Aspekt ist es bevorzugt, dass die Schichten des erfindungsgemäßen Rohlings auf Basis von Lithiumsilikat-Glas, Lithiumsilikat-Glas mit Keimen oder Lithiummetasilikat-Glaskeramik sind oder aus diesen bestehen. Einem derartigen Rohling kann aufgrund seiner relativ geringen Festigkeit besonders einfach durch maschinelle Bearbeitung die Form der gewünschten Dentalrestauration verliehen werden. Gemäß einer alternativen Ausführungsform sind die Schichten des erfindungsgemäßen Rohlings auf Basis von Lithiumdisilikat-Glaskeramik.

Besonders bevorzugt enthalten das Lithiumsilikat-Glas, das Lithiumsilikat-Glas mit Keimen, die Lithiummetasilikat-Glaskeramik oder die Lithiumdisilikat-Glaskeramik mindestens eine und vorzugsweise alle der folgenden Komponenten in den angegebenen Mengen, wobei die Mengen der Komponenten als Oxide berechnet werden, so wie es bei Gläsern und Glaskeramiken üblich ist:

| Komponente | Gew.-% |
|---|---|
| SiO₂ | 64,0 bis 73,0 |
| Li₂O | 12,0 bis 18,0 |
| K₂O | 1,0 bis 5,0 |
| Al₂O₃ | 0,5 bis 5,0 |
| P₂O₅ | 1,0 bis 7,0. |

Weiterhin kann das Lithiumsilikat-Glas, das Lithiumsilikat-Glas mit Keimen, die Lithiummetasilikat-Glaskeramik oder die Lithiumdisilikat-Glaskeramik vorzugsweise mindestens eine und insbesondere alle der folgenden Komponenten in den angegebenen Mengen enthalten:

| Komponente | Gew.-% |
|---|---|
| ZrO₂ | 0 bis 1,0, wie etwa 0,1 |
| ZnO | 0 bis 6,0 |
| Na₂O | 0 bis 2,0 |
| Me^{II}O | 0 bis 5,0, |

wobei Me^{II}O ein zweiwertiges Oxid ist, das insbesondere ausgewählt ist aus MgO, CaO und/oder SrO.

Weitere bevorzugte Zusammensetzungen für ein Lithiumsilikat-Glas, ein Lithiumsilikat-Glas mit Keimen, eine Lithiummetasilikat-Glaskeramik oder eine Lithiumdisilikat-Glaskeramik werden in der EP 1 505 041 A1 und der EP 1 688 398 A1 beschrieben.

Insbesondere sind solche Zusammensetzungen bevorzugt, bei denen sich die Menge an Oxiden von Elementen mit einer Ordnungszahl von 19 oder höher in der ersten Schicht um nicht mehr als 2 Gew.-%, vorzugsweise nicht um mehr als 1,5 Gew.-%, von der Menge an Oxiden von Elementen mit einer Ordnungszahl von 19 oder höher in der zweiten Schicht unterscheidet.

Es wurde überraschenderweise gefunden, dass es durch die oben genannten bevorzugten Zusammensetzungen und insbesondere durch die oben genannte bevorzugte Bedingung hinsichtlich der Menge an Oxiden mit einer Ordnungszahl von 19 oder größer möglich ist, eine erste und zweite Schicht bereitzustellen, die trotz ihrer unterschiedlichen Farbe nahezu identische Brechungsindizes aufweisen. Auf diese Art und Weise ist es möglich, einen Rohling und später auch Zahnersatz bereitzustellen, bei dem keine störende Grenzfläche zwischen Schichten sichtbar ist.

Das Lithiumsilikat-Glas wird üblicherweise durch Erschmelzen geeigneter Ausgangsstoffe hergestellt. Dieses Glas kann durch Wärmebehandlung in das Lithiumsilikat-Glas mit Keimen umgewandelt werden. Bei den Keimen handelt es sich um solche, die für die Kristallisation von Lithiummetasilikat und/oder Lithiumdisilikat geeignet sind. Das Lithiumsilikat-Glas mit Keimen kann durch Wärmebehandlung in die Lithiummetasilikat-Glaskeramik überführt werden.

Es ist schließlich möglich, die Lithiummetasilikat-Glaskeramik durch weitere Wärmebehandlung in hochfeste Lithiumdisilikat-Glaskeramik umzuwandeln. Daher sind das Lithiumsilikat-Glas, das Lithiumsilikat-Glas mit Keimen und die Lithiummetasilikat-Glaskeramik Vorläufer der Lithiumdisilikat-Glaskeramik.

Weiter ist ein Rohling bevorzugt, bei dem die Lithiummetasilikat-Glaskeramik als Hauptkristallphase Lithiummetasilikat enthält und insbesondere mehr als 5 Vol.-%, bevorzugt mehr als 10 Vol.-% und besonders bevorzugt mehr als 20 Vol.-% an Lithiummetasilikat-Kristallen enthält. Dabei bezeichnet der Begriff "Hauptkristallphase" die Kristallphase, die gegenüber anderen Kristallphasen den höchsten Volumenanteil hat.

In einer weiteren Ausführungsform ist ein Rohling auf Basis einer Glaskeramik bevorzugt, der neben einer Lithiumsilikat-Kristallphase, insbesondere einer Lithiummetasilikat- oder Lithiumdisilikat-Kristallphase, eine weitere Kristallphase, vorzugsweise eine SiO₂-Kristallphase, wie z.B. Tiefquarz, enthält. Besonders bevorzugt enthält ein derartiger Rohling mindestens eine und vorzugsweise alle der folgenden Komponenten in den angegebenen Mengen:

| Komponente | Gew.-% |
|---|---|
| SiO₂ | 59,0 bis 79,0 oder 68,0 bis 79,0 |
| Li₂O | 8,0 bis 15,0 |
| P₂O₅ | 0 bis 9,0 |
| Me^{I}₂O | 1,0 bis 8,0 |
| Me^{II}O | 1,0 bis 9,0 |
| Me^{III}₂O₃ | 1,0 bis 8,0, |

wobei Me^{I}₂O ausgewählt ist aus der Gruppe von K₂O, Na₂O, Rb₂O, Cs₂O und Mischungen davon, Me^{II}O ausgewählt ist aus der Gruppe von CaO, MgO, SrO, ZnO und Mischungen davon und Me^{III}₂O₃ ausgewählt ist aus der Gruppe von Al₂O₃, B₂O₃, Y₂O₃, La₂O₃, Ga₂O₃, In₂O₃ und Mischungen davon.

Bei derartigen Glaskeramiken ist das Molverhältnis von SiO₂ zu Li₂O vorzugsweise im Bereich von 2,2 bis 3,8. Des Weiteren ist es bevorzugt, dass der Gehalt an Lithiumdisilikat in der Glaskeramik mehr als 20 Gew.-%, vorzugsweise 25 bis 55 Gew.-%, beträgt. Auch ist es bevorzugt, dass der Gehalt an Tiefquarz 0,2 bis 28 Gew.-% beträgt. Weitere bevorzugte Glaskeramiken, die neben einer Lithiumsilikat-Kristallphase eine Tiefquarz-Kristallphase enthalten, werden in der EP 3 315 641 beschrieben.

In einer bevorzugten Ausführungsform weist der erfindungsgemäße Rohling monolithische Schichten von Lithiumsilikat-Glas, monolithische Schichten von Lithiumsilikat-Glas mit Keimen, monolithische Schichten von Lithiummetasilikat-Glaskeramik oder monolithische Schichten von Lithiumdisilikat-Glaskeramik auf.

Der Begriff "monolithisch" bezeichnet Schichten, die kontinuierlich sind und sich damit von diskontinuierlichen Schichten, wie Schichten von Teilchen, z.B. Pulver- oder Granulatschichten, unterscheiden. Die erfindungsgemäß verwendeten monolithischen Schichten können auch als Massivschichten der Gläser und Glaskeramiken bezeichnet werden.

Das Vorliegen von monolithischen Schichten in dem Rohling gemäß dem ersten Aspekt der Erfindung ist dafür mit verantwortlich, dass seine Umwandlung zur gewünschten hochfesten Dentalrestauration durch Wärmebehandlung ohne wesentliche Schrumpfung möglich ist. Die demgegenüber bei herkömmlichen Rohlingen vorliegenden diskontinuierlichen Schichten, wie gepresste Schichten aus Pulvern oder Granulaten, müssen noch dichtgesintert werden, um die endgültige Dentalrestauration zu ergeben. Dieses Dichtsintern führt jedoch zu einer erheblichen Schrumpfung. Zur Erzeugung von passgenauen Dentalrestaurationen muss daher zunächst eine vergrößerte Form der Restauration erzeugt werden und diese vergrößerte Form wird dann dichtgesintert. Eine solche Verfahrensweise ist jedoch aufwendig und fehleranfällig. Sie erfordert auch zunächst die genaue Bestimmung des jeweils zu wählenden Vergrößerungsfaktors, der unter anderem von den genauen Sinterbedingungen und der Art des eingesetzten Rohlings abhängt.

Gemäß einem zweiten Aspekt ist es bevorzugt, dass die Schichten des erfindungsgemäßen Rohlings auf Basis von Siliciumdioxid-Glas, Siliciumdioxid-Glas mit Keimen oder Siliciumdioxid-Glaskeramik sind oder aus diesen bestehen. Insbesondere ein Rohling, bei dem die Schichten auf Basis von Siliciumdioxid-Glaskeramik sind oder aus diesen bestehen, ist bevorzugt. Ein derartiger Rohling kann selbst in einer vollständig kristallisierten Form verhältnismäßig einfach durch maschinelle Bearbeitung die Form der gewünschten Dentalrestauration verliehen werden. Eine thermische Behandlung nach der maschinellen Bearbeitung ist in dem Fall nicht mehr notwendig, wodurch ein derartiger Rohling besonders vorteilhaft ist.

Besonders bevorzugt enthalten das Siliciumdioxid-Glas, das Siliciumdioxid-Glas mit Keimen oder die Siliciumdioxid-Glaskeramik mindestens eine und vorzugsweise alle der folgenden Komponenten in den angegebenen Mengen:

| Komponente | Gew.-% |
|---|---|
| SiO₂ | 58,0 bis 92,0 |
| Li₂O | 2,0 bis 10,0 |
| Me^{I}₂O | 0 bis 13,0 |
| Me^{II}O | 0 bis 11,0 |
| Me^{III}₂O₃ | 0 bis 10,0 |
| Me^{IV}O₂ | 0 bis 21,0 |
| P₂O₅ | 0 bis 7,0 |
| Me^{V}₂O₅ | 0 bis 6,0 |
| Me^{VI}O₃ | 0 bis 6,0 |
| Fluor | 0 bis 5,0, |

wobei Me^{I}₂O insbesondere ausgewählt ist aus Na₂O, K₂O, Rb₂O und/oder Cs₂O; Me^{II}O insbesondere ausgewählt ist aus MgO, CaO, SrO und/oder ZnO; Me^{III}₂O₃ insbesondere ausgewählt ist aus Al₂O₃, B₂O₃, Y₂O₃, La₂O₃, Ga₃O₃ und/oder In₂O₃; Me^{IV}O₂ insbesondere ausgewählt ist aus ZrO₂, GeO₂, CeO₂, TiO₂ und/oder SnO₂; Me^{V}₂O₅ insbesondere ausgewählt ist aus V₂O₅, Ta₂O₅ und/oder Nb₂O₅; und Me^{VI}O₃ insbesondere ausgewählt ist aus WO₃ und/oder MoO₃.

Besonders bevorzugt enthält die Siliciumdioxid-Glaskeramik SiO₂, insbesondere Tiefquarz, Cristoballit oder eine Mischung von diesen, als Hauptkristallphase.

Weitere bevorzugte Siliciumdioxid-Gläser, Siliciumdioxid-Gläser mit Keimen oder Siliciumdioxid-Glaskeramiken werden in der WO 2015/173394 A1 beschrieben.

Der Rohling gemäß dem zweiten Aspekt weist vorzugsweise monolithische Schichten von Siliciumdioxid-Glas, monolithische Schichten von Siliciumdioxid-Glas mit Keimen oder monolithische Schichten von Siliciumdioxid-Glaskeramik auf.

Gemäß einem dritten Aspekt ist es bevorzugt, dass die erste und zweite Schicht des erfindungsgemäßen Rohlings ungesintertes Zirkonoxid oder vorgesintertes Zirkonoxid enthalten.

Besonders bevorzugt enthält ein Rohling gemäß des dritten Aspekts mindestens eine und vorzugsweise alle der folgenden Komponenten in den angegebenen Mengen:

| | Einheit | erste Schicht | zweite Schicht |
|---|---|---|---|
| ZrO₂+HfO₂ | Gew.-% | 86,77 - 96,50 | 86,28 - 93,00 |
| Y₂O₃ | Gew.-% | 3,5 - 8,0 | 7,0 - 10,0 |
| Al₂O₃ | Gew.-% | 0,0 - 1,0 | 0,0 - 0,1 |
| SiO₂ | Gew.-% | ≤ 0,02 | ≤ 0,02 |
| Na₂O | Gew.-% | ≤ 0,04 | ≤ 0,04 |
| TiO₂ | Gew.-% | ≤ 0,10 | ≤ 0,10 |
| CaO | Gew.-% | ≤ 0,10 | ≤ 0,10 |
| Fe₂O₃ | Gew.-% | 0,001 - 0,200 | 0,002 - 0,100 |
| Mn₃O₃ | Gew.-% | 0,000 - 0,001 | 0,000 - 0,001 |
| Cr₂O₃ | Gew.-% | 0,00 - 0,01 | 0,000 - 0,005 |
| Pr₂O₃ | Gew.-% | 0,000 - 0,003 | 0,00 - 0,002 |
| Tb₂O₃ | Gew.-% | 0,00 - 0,02 | 0,000 - 0,015 |
| Er₂O₃ | Gew.-% | 0,0 - 1,0 | 0,0 - 0,5 |
| CoO | Gew.-% | 0,0 - 0,04 | 0,0 - 0,04 |
| NiO | Gew.-% | ≤ 0,10 | ≤ 0,10 |
| Yb₂O₃ | Gew.-% | ≤ 1,0 | ≤ 1,0 |
| La₂O₃ | Gew.-% | ≤ 1,0 | ≤ 1,0 |
| MgO | Gew.-% | ≤ 0,10 | ≤ 0,10 |
| weitere Oxide | Gew.-% | ≤ 0,50 | ≤ 0,50 |

Die erfindungsgemäßen Rohlinge, einschließlich der erfindungsgemäßen Rohlinge des ersten, zweiten und dritten Aspekts, liegen vorzugsweise in Form von Blöcken, Quadern, Scheiben oder Zylindern, wie z.B. Kreiszylindern oder Zylindern mit elliptischer Grundfläche, vor. In diesen Formen können sie besonders einfach zu den gewünschten Dentalrestaurationen weiterverarbeitet werden. Besonders bevorzugt liegen die erfindungsgemäßen Rohlinge in Form von Blöcken vor.

In einer weiteren bevorzugten Ausführungsform weisen die erfindungsgemäßen Rohlinge einen Halter für die Befestigung in einer Bearbeitungsvorrichtung auf. Der Halter gestattet die Befestigung der Rohlinge in einer Bearbeitungsvorrichtung, wie insbesondere einer Fräs- oder Schleifvorrichtung. Der Halter hat üblicherweise die Form eines Stifts, und der Halter besteht vorzugsweise aus Metall oder Kunststoff.

Die Erfindung betrifft ebenfalls ein Verfahren zur Herstellung der erfindungsgemäßen Rohlinge.

Das Verfahren zur Herstellung des erfindungsgemäßen Rohlings gemäß dem ersten Aspekt, d.h. eines Rohlings mit Schichten, insbesondere monolithischen Schichten, von Lithiumsilikat-Glas, Lithiumsilikat-Glas mit Keimen oder Lithiummetasilikat-Glaskeramik, oder dem zweiten Aspekt, d.h. eines Rohlings mit Schichten, insbesondere monolithischen Schichten von Siliciumdioxid-Glas, Siliciumdioxid-Glas mit Keimen oder Siliciumdioxid-Glaskeramik, zeichnet sich dadurch aus, dass
(a1) eine erste Schicht von Lithiumsilikat-Glas, Lithiumsilikat-Glas mit Keimen, Lithiummetasilikat-Glaskeramik, Siliciumdioxid-Glas, Siliciumdioxid-Glas mit Keimen oder Siliciumdioxid-Glaskeramik mit einer Viskosität von mindestens 6,6 Pa·s in einer Form bereitgestellt wird,
(b1) die Oberfläche der ersten Schicht geformt wird, um den gewünschten Verlauf der Grenzfläche der ersten und zweiten Schicht des Rohlings bereitzustellen, und
(c1) eine zweite Schicht von Lithiumsilikat-Glas, Lithiumsilikat-Glas mit Keimen, Lithiummetasilikat-Glaskeramik, Siliciumdioxid-Glas, Siliciumdioxid-Glas mit Keimen oder Siliciumdioxid-Glaskeramik auf die Oberfläche der ersten Schicht aufgebracht wird.

Besonders bevorzugt wird in Schritt (a1) eine erste Schicht eines Glases, insbesondere von Lithiumsilikat-Glas oder Siliciumdioxid-Glas, in der Form bereitgestellt.

Die in Schritt (b1) durchgeführte Formung der Oberfläche der ersten Schicht kann vorzugsweise durch Pressen mit einem strukturierten Oberstempel, z.B. einem Stempel aus Graphit, erreicht werden. Aufgrund der relativ geringen Festigkeit des vorzugsweise in Schritt (a1) eingesetzten Glases in Schritt (b1) ist ein geringer Pressdruck von z.B. weniger als 10 MPa ausreichend, um die gewünschte Formgebung zu erzielen.

Des Weiteren ist es bevorzugt, das in Schritt (c1) eine zweite Schicht eines Glases, insbesondere von Lithiumsilikat-Glas oder Siliciumdioxid-Glas, auf die geformte erste Schicht aufgebracht wird. Dieses Aufbringen kann beispielsweise mittels Gießen des Glases erfolgen.

Ferner ist es bevorzugt, dass der Rohling zwischen Formgebung der Grenzfläche und Überschichtung mit dem Material der zweiten Schicht keiner Wärmebehandlung zur Ausbildung von kristallinen Phasen unterzogen wird. Vielmehr ist es bevorzugt, dass im Anschluss an Schritt (c1) der komplette Rohling einer Wärmebehandlung unterzogen wird, z.B. zur Ausbildung von Kristallphasen, wie z.B. einer Lithiummetasilikat-Glaskeramik oder einer Siliciumdioxid-Glaskeramik.

In einem alternativen Verfahren kann ein Rohling gemäß dem ersten oder zweiten Aspekt durch schrittweises Befüllen von pulverförmigen Ausgangsmaterials in eine Pressmatrize hergestellt werden.

Das Verfahren zur Herstellung des erfindungsgemäßen Rohlings gemäß dem dritten Aspekt, d.h. eines Rohlings mit Schichten von Zirkonoxid zeichnet sich dadurch aus, dass
(a2) eine erste Schicht von ungesintertem oder dispergiertem Zirkonoxid in einer Form bereitgestellt wird,
(b2) die Oberfläche der ersten Schicht geformt wird, um den gewünschten Verlauf der Grenzfläche der ersten und zweiten Schicht des Rohlings bereitzustellen, und
(c2) eine zweite Schicht von ungesintertem oder dispergiertem Zirkonoxid auf die Oberfläche der ersten Schicht aufgebracht wird.

Der Begriff "dispergiert" bezeichnet dabei Zirkonoxid, das in einer Suspension in einem flüssigen Medium, wie wässrigem oder organischem Lösungsmittel, homogen verteilt ist. Dabei ist die Viskosität der Suspension vorzugsweise so hoch, dass nach der Formgebung in Schritt (b2) die geformte Form der Oberfläche erhalten bleibt.

Besonders bevorzugt wird im Anschluss an Schritt (c2) der komplette Rohling einer Wärmebehandlung unterzogen, um einen vorgesinterten Rohling bereitzustellen und so die Verarbeitbarkeit und Präzision bei einer späteren maschinellen Bearbeitung zur Herstellung von Dentalrestaurationen zu verbessern.

Die erfindungsgemäßen Rohlinge sind aufgrund ihrer Eigenschaften besonders geeignet, zu Dentalrestaurationen weiterverarbeitet zu werden.

Die Erfindung betrifft daher auch ein Verfahren zur Herstellung von Dentalrestaurationen, bei dem
(d1) einem Rohling gemäß dem ersten Aspekt der Erfindung durch maschinelle Bearbeitung die Form der Dentalrestauration gegeben wird,
(e1) mindestens eine Wärmebehandlung durchgeführt wird, um das Lithiumsilikat-Glas, das Lithiumsilikat-Glas mit Keimen oder die Lithiummetasilikat-Glaskeramik in Lithiumdisilikat-Glaskeramik umzuwandeln, und
(f1) gegebenenfalls die Oberfläche der erhaltenen Dentalrestauration endbehandelt wird
oder in einer alternativen Ausführungsform
(d2) einem Rohling gemäß dem zweiten Aspekt der Erfindung durch maschinelle Bearbeitung die Form der Dentalrestauration gegeben wird,
(e2) gegebenenfalls eine Wärmebehandlung durchgeführt wird, um das Siliciumdioxid-Glas oder das Siliciumdioxid-Glas mit Keimen in eine Siliciumdioxid-Glaskeramik umzuwandeln oder den Kristallgehalt einer Siliciumdioxid-Glaskeramik zu erhöhen, und
(f2) gegebenenfalls die Oberfläche der erhaltenen Dentalrestauration endbehandelt wird
oder in einer alternativen Ausführungsform
(d3) einem Rohling gemäß dem dritten Aspekt der Erfindung durch maschinelle Bearbeitung die Form der Dentalrestauration gegeben wird,
(e3) mindestens eine Wärmebehandlung durchgeführt wird, um das ungesinterte oder vorgesinterte Zirkonoxid in dichtgesintertes Zirkonoxid umzuwandeln, und
(f3) gegebenenfalls die Oberfläche der erhaltenen Dentalrestauration endbehandelt wird.

Aus den erfindungsgemäßen Rohlingen können in einfacher Weise durch maschinelle Bearbeitung die gewünscht geformten Dentalrestaurationen herausgearbeitet werden. Es werden hierfür gemäß dem ersten Aspekt der Erfindung insbesondere Rohlinge mit Schichten von Lithiumsilikat-Glas mit Keimen oder Lithiummetasilikat-Glaskeramik verwendet (Schritt (d1)) oder gemäß dem zweiten Aspekt insbesondere Rohlinge mit Schichten von Siliciumdioxid-Glaskeramik (Schritt (d2)) oder gemäß dem dritten Aspekt der Erfindung insbesondere Rohlinge mit Schichten von vorgesintertem Zirkonoxid verwendet (Schritt (d3)).

Die maschinelle Bearbeitung erfolgt üblicherweise durch materialabtragende Verfahren und insbesondere durch Fräsen und/oder Schleifen. Es ist bevorzugt, dass die maschinelle Bearbeitung mit computer-gesteuerten Fräs- und/oder Schleifvorrichtungen erfolgt. Besonders bevorzugt erfolgt die maschinelle Bearbeitung im Rahmen eines CAD/CAM-Verfahrens.

In Schritt (e1) wird der Rohling einer Wärmebehandlung unterzogen, um die gesteuerte Kristallisation von Lithiumdisilikat und damit die Bildung von Lithiumdisilikat-Glaskeramik herbeizuführen. Die Wärmebehandlung findet insbesondere bei einer Temperatur von 750 bis 950°C und bevorzugt 800 bis 900°C statt. Die Wärmebehandlung wird insbesondere für eine Dauer von 1 bis 30 min, bevorzugt 2 bis 15 min, durchgeführt.

In Schritt (e2) wird der Rohling optional einer Wärmebehandlung unterzogen. Im Fall eines Rohlings auf Basis von Siliciumdioxid-Glaskeramik ist es jedoch bevorzugt, dass keine Wärmebehandlung gemäß (e2) durchgeführt. Ein solches Verfahren ist besonders einfach und kostengünstig und daher besonders bevorzugt.

In Schritt (e3) wird der Rohling einer Wärmebehandlung unterzogen, um die Bildung von dichtgesinterter Zirkonoxid-Keramik herbeizuführen. Die Wärmebehandlung findet insbesondere bei einer Temperatur von 1050 bis 1600°C und bevorzugt 1450 bis 1550°C statt. Die Wärmebehandlung wird insbesondere für eine Dauer von 0 bis 240 min, bevorzugt 5 bis 180 min, besonders bevorzugt 30 bis 120 min, durchgeführt, wobei sich der Begriff "Dauer" auf die Haltezeit der Maximaltemperatur bezieht.

Es liegen nach Durchführung der Stufen (e1) bzw. (e2) bzw. (e3) Dentalrestaurationen mit Schichten von Lithiumdisilikat-Glaskeramik, Siliciumdioxid-Glaskeramik oder Zirkonoxid-Keramik vor, die über ausgezeichnete mechanische Eigenschaften und eine hohe chemische Stabilität verfügen. Darüber hinaus gestatten sie durch die mehreren sich in der Farbe unterscheidenden Schichten eine ausgezeichnete Nachahmung der optischen Eigenschaften von natürlichem Zahnmaterial, z.B. von Farbverläufen vom Dentin zur Schneide. Schließlich können die Restaurationen mit Hilfe der Schritte (d1) bis (f1) bzw. (d2) bis (f2) auch ohne wesentliche Schrumpfung aus den erfindungsgemäßen Rohlingen erzeugt werden. Das beruht insbesondere darauf, dass die erfindungsgemäßen Rohlinge gemäß dem ersten und zweiten Aspekt monolithische Schichten und nicht diskontinuierliche Schichten, wie Pulver- oder Granulatschichten, aufweisen, wodurch auf eine Sinterung nach der Formgebung und eine damit einhergehende Schrumpfung verzichtet werden kann. Durch Einsatz der erfindungsgemäßen Rohlinge können daher in besonders einfacher Weise Dentalrestaurationen mit genau den gewünschten Abmessungen hergestellt werden. Bei erfindungsgemäßen Rohlingen gemäß dem dritten Aspekt kann das Problem des in Schritt (e3) auftretenden Sinterschrumpfes dadurch gelöst werden, dass die erste und die zweite Schicht eine im Wesentlichen gleiche Gesamtschwindung während Schritt (e3) aufweisen. Eine derartige Einstellung der Gesamtschwindung kann dadurch erfolgen, dass die Ausgangs- und Enddichte vor und nach der Wärmebehandlung in den beiden Schichten jeweils gleich sind. Insbesondere kann der Beginn des Sinterns der einzelnen Schichten durch deren Zusammensetzung, insbesondere mittels Zugabe von Sinteraktivatoren und/oder -inhibitoren, aneinander angepasst werden. Aufgrund des schrägen Verlaufs der Grenzfläche der ersten und der zweiten Schicht wird die Passung der herzustellenden Dentalrestauration insbesondere von der Passung und somit vom Sinterschrumpf der ersten Schicht des erfindungsgemäßen Rohlings bestimmt, welche die Dentinschicht imitiert, und wird weniger stark von etwaigen unterschiedlichen Sinterschrumpfverhalten in den einzelnen Schichten beeinflusst, wie es bei herkömmlichen Rohlingen mit horizontaler Schichtabfolge der Fall wäre.

Die erfindungsgemäß hergestellten Dentalrestaurationen sind bevorzugt ausgewählt aus Kronen, Abutments, Abutmentkronen, Inlays, Onlays, Veneers, Schalen und Brücken sowie Überwürfen für mehrteilige Restaurationsgerüste, die z.B. aus Oxidkeramik, Metallen oder Dentallegierungen bestehen können.

In den optionalen Stufen (f1), (f2) und (f3) kann die Oberfläche der Dentalrestauration noch endbehandelt werden. Dabei ist es insbesondere möglich, noch einen Glanzbrand bei einer Temperatur von 700 bis 850°C durchzuführen oder die Restauration zu polieren. Darüber hinaus kann auch eine Schichtmasse aus Gläsern und/oder Glaskeramiken aufgebracht werden.

Aufgrund der geschilderten besonderen Eigenschaften der erfindungsgemäßen Rohlinge eignen sich diese insbesondere zur Erzeugung von Dentalrestaurationen. Die Erfindung betrifft daher auch die Verwendung der Rohlinge zur Herstellung von Dentalrestaurationen und insbesondere von Kronen, Abutments, Abutmentkronen, Inlays, Onlays, Veneers, Schalen und Brücken sowie Überwürfen. Besonders bevorzugt ist die Verwendung der erfindungsgemäßen Rohlinge zur Herstellung von Zahnersatz im Frontzahnbereich, wie z.B. einer Frontzahnkrone.

## Patentansprüche

1. Rohling (1) für dentale Zwecke, der eine erste und eine zweite Schicht (3, 5) aufweist, die unabhängig voneinander auf Basis von
Glas,
Glaskeramik oder
Keramik
sind, wobei die erste Schicht und die zweite Schicht sich in der Farbe unterscheiden und eine Grenzfläche (7) bilden, wobei die Grenzfläche schräg verläuft, wobei der Rohling eine Rotationsachse und eine Einschubachse aufweist, wobei die Rotationsachse die Achse bezeichnet, um die der Rohling bei der maschinellen Bearbeitung zur Formgebung der gewünschten Dentalrestauration gedreht wird, und die Einschubachse die Achse bezeichnet, die in zervikal-inzisaler Richtung verläuft,
wobei in einer ersten Schnittebene (15) durch den Rohling, die parallel zur Einschubachse (8) des Rohlings verläuft, die Grenzfläche (7) einen Winkel von 10 bis 70°, vorzugsweise 10 bis 60°, zur Rotationsachse des Rohlings aufweist,
wobei der Winkel zwischen der Einschubachse (8) und der Rotationsachse (9) des Rohlings in der ersten Schnittebene 70 bis 110^{c} beträgt.

2. Rohling nach Anspruch 1, bei dem in einer ersten Schnittebene durch den Rohling, die parallel zur Einschubachse des Rohlings verläuft, die Grenzfläche nicht senkrecht zur Einschubachse verläuft.

3. Rohling nach Anspruch 1 oder 2, bei dem die Grenzfläche in der ersten Schnittebene im Wesentlichen geradlinig verläuft,
wobei die Grenzfläche in der ersten Schnittebene vorzugsweise einen Winkel von 20 bis 80°C, besonders bevorzugt von 30 bis 80°, zur Einschubachse aufweist
oder
bei dem die Grenzfläche in der ersten Schnittebene bogenförmig verläuft und die Ausgleichsgerade durch die bogenförmige Grenzfläche in der ersten Schnittebene vorzugsweise einen Winkel von 20 bis 80°, besonders bevorzugt 30 bis 80°, zur Einschubachse aufweist.

4. Rohling nach einem der Ansprüche 1 bis 3, bei dem der Winkel zwischen der Einschubachse und der Rotationsachse des Rohlings in der ersten Schnittebene 80 bis 100° und besonders bevorzugt etwa 90° beträgt.

5. Rohling nach einem der Ansprüche 1 bis 4, bei dem die Grenzfläche (7) in einer zweiten Schnittebene (23) durch den Rohling, die senkrecht zur ersten Schnittebene verläuft, bogenförmig verläuft, wobei die Grenzfläche in der zweiten Schnittebene vorzugsweise konvex gewölbt verläuft.

6. Rohling nach Anspruch 5, bei dem die Grenzfläche in der zweiten Schnittebene eine Mamelonstruktur (25) aufweist, wobei die Mamelonstruktur vorzugsweise Einkerbungen aufweist, deren Tiefe bis zu 2 mm, vorzugsweise 0,1 bis 0,5 mm, beträgt.

7. Rohling nach einem der vorhergehenden Ansprüche, bei dem die erste Schicht einen Brechungsindex aufweist, der sich um nicht mehr als 0,1 von dem Brechungsindex der zweiten Schicht unterscheidet
und/oder
der eine Markierung aufweist, die von einer CAD/CAM-Vorrichtung erkannt und mit der die Lage der Grenzfläche mit einer Genauigkeit von insbesondere 0,1 mm bestimmt werden kann.

8. Rohling nach einem der vorhergehenden Ansprüche, bei dem das Glas, die Glaskeramik oder die Keramik ausgewählt sind aus Lithiumsilikat-Glas, Lithiumsilikat-Glaskeramik, Siliciumdioxid-Glas, Siliciumdioxid-Glaskeramik und/oder Zirkonoxid.

9. Rohling nach Anspruch 8, bei dem die erste und zweite Schicht unabhängig voneinander auf Basis von
Lithiumsilikat-Glas,
Lithiumsilikat-Glas mit Keimen oder
Lithiummetasilikat-Glaskeramik
sind, wobei das Lithiumsilikat-Glas, das Lithiumsilikat-Glas mit Keimen oder die Lithiummetasilikat-Glaskeramik mindestens eine und vorzugsweise alle der folgenden Komponenten in den angegebenen Mengen enthalten:
| Komponente | Gew.-% |
|---|---|
| SiO₂ | 64,0 bis 73,0 |
| Li₂O | 12,0 bis 18,0 |
| K₂O | 1,0 bis 5,0 |
| Al₂O₃ | 0,5 bis 5,0 |
| P₂O₅ | 1,0 bis 7,0, |
wobei sich die Menge an Oxiden von Elementen mit einer Ordnungszahl von 19 oder höher in der ersten Schicht vorzugsweise um nicht mehr als 2 Gew.-% von der Menge an Oxiden von Elementen mit einer Ordnungszahl von 19 oder höher in der zweiten Schicht unterscheidet.

10. Rohling nach Anspruch 8, bei dem die erste und zweite Schicht unabhängig voneinander auf Basis von
Siliciumdioxid-Glas,
Siliciumdioxid-Glas mit Keimen oder
Siliciumdioxid-Glaskeramik
sind, wobei das Siliciumdioxid-Glas, das Siliciumdioxid-Glas mit Keimen oder die Siliciumdioxid-Glaskeramik mindestens eine und vorzugsweise alle der folgenden Komponenten in den angegebenen Mengen enthalten:
| Komponente | Gew.-% |
|---|---|
| SiO₂ | 58,0 bis 92,0 |
| Li₂O | 2,0 bis 10,0 |
| Me^{I}₂O | 0 bis 13,0 |
| Me^{II}O | 0 bis 11,0 |
| Me^{III}₂O₃ | 0 bis 10,0 |
| Me^{IV}O₂ | 0 bis 21,0 |
| P₂O₅ | 0 bis 7,0 |
| Me^{V}₂O₅ | 0 bis 6,0 |
| Me^{VI}O₃ | 0 bis 6,0 |
| Fluor | 0 bis 5,0, |
wobei Me^{I}₂O insbesondere ausgewählt ist aus Na₂O, K₂O, Rb₂O und/oder Cs₂O; Me^{II}O insbesondere ausgewählt ist aus MgO, CaO, SrO und/oder ZnO; Me^{III}₂O₃ insbesondere ausgewählt ist aus Al₂O₃, B₂O₃, Y₂O₃, La₂O₃, Ga₂O₃ und/oder In₂O₃; Me^{IV}O₂ insbesondere ausgewählt ist aus ZrO₂, GeO₂, CeO₂, TiO₂ und/oder SnO₂; Me^{V}₂O₅ insbesondere ausgewählt ist aus V₂O₅, Ta₂O₅ und/oder Nb₂O₅; und Me^{VI}O₃ insbesondere ausgewählt ist aus WO₃ und/oder MoO₃.

11. Rohling nach Anspruch 9 oder 10, bei dem die erste und zweite Schicht monolithisch sind.

12. Rohling nach Anspruch 8, bei dem die erste und zweite Schicht
unabhängig voneinander
ungesintertes Zirkonoxid oder
vorgesintertes Zirkonoxid
enthalten.

13. Rohling nach einem der vorhergehenden Ansprüche, der einen Halter (29) für eine Bearbeitungsvorrichtung aufweist, wobei der Halter insbesondere aus Metall oder Kunststoff besteht.

14. Verfahren zur Herstellung eines Rohlings gemäß einem der Ansprüche 1 bis 13, bei dem
(a) eine erste Schicht auf Basis von Glas, Glaskeramik oder Keramik bereitgestellt wird,
(b) die Oberfläche der ersten Schicht geformt wird, um den gewünschten Verlauf der Grenzfläche der ersten und zweiten Schicht des Rohlings bereitzustellen, und
(c) eine zweite Schicht auf Basis von Glas, Glaskeramik oder Keramik auf die Oberfläche der ersten Schicht aufgebracht wird.

15. Verfahren zur Herstellung eines Rohlings mit Schichten von Lithiumsilikat-Glas, Lithiumsilikat-Glas mit Keimen, Lithiummetasilikat-Glaskeramik, Siliciumdioxid-Glas, Siliciumdioxid-Glas mit Keimen oder Siliciumdioxid-Glaskeramik gemäß einem der Ansprüche 9 bis 11, bei dem
(a1) eine erste Schicht von Lithiumsilikat-Glas, Lithiumsilikat-Glas mit Keimen, Lithiummetasilikat-Glaskeramik, Siliciumdioxid-Glas, Siliciumdioxid-Glas mit Keimen oder Siliciumdioxid-Glaskeramik mit einer Viskosität von mindestens 6,6 Pa·s in einer Form bereitgestellt wird,
(b1) die Oberfläche der ersten Schicht geformt wird, um den gewünschten Verlauf der Grenzfläche der ersten und zweiten Schicht des Rohlings bereitzustellen, und
(c1) eine zweite Schicht von Lithiumsilikat-Glas, Lithiumsilikat-Glas mit Keimen, Lithiummetasilikat-Glaskeramik, Siliciumdioxid-Glas, Siliciumdioxid-Glas mit Keimen oder Siliciumdioxid-Glaskeramik auf die Oberfläche der ersten Schicht aufgebracht wird.

16. Verfahren zur Herstellung eines Rohlings mit Schichten von Zirkonoxid gemäß Anspruch 12, bei dem
(a2) eine erste Schicht von ungesintertem oder dispergiertem Zirkonoxid in einer Form bereitgestellt wird,
(b2) die Oberfläche der ersten Schicht geformt wird, um den gewünschten Verlauf der Grenzfläche der ersten und zweiten Schicht des Rohlings bereitzustellen, und
(c2) eine zweite Schicht von ungesintertem oder dispergiertem Zirkonoxid auf die Oberfläche der ersten Schicht aufgebracht wird.

17. Verfahren zur Herstellung einer Dentalrestauration, bei dem
(d) einem Rohling gemäß einem der Ansprüche 1 bis 13 durch maschinelle Bearbeitung die Form der Dentalrestauration gegeben wird, wobei die maschinelle Bearbeitung vorzugsweise mit computergesteuerten Fräs- und/oder Schleifvorrichtungen erfolgt,
(e) gegebenenfalls mindestens eine Wärmebehandlung durchgeführt wird, und
(f) gegebenenfalls die Oberfläche der erhaltenen Dentalrestauration endbehandelt wird.

18. Verfahren nach Anspruch 17, bei dem
(d1) einem Rohling gemäß Anspruch 9 oder 11 durch maschinelle Bearbeitung die Form der Dentalrestauration gegeben wird, wobei die maschinelle Bearbeitung vorzugsweise mit computergesteuerten Fräs- und/oder Schleifvorrichtungen erfolgt,
(e1) mindestens eine Wärmebehandlung durchgeführt wird, um das Lithiumsilikat-Glas, das Lithiumsilikat-Glas mit Keimen oder die Lithiummetasilikat-Glaskeramik in Lithiumdisilikat-Glaskeramik umzuwandeln, und
(f1) gegebenenfalls die Oberfläche der erhaltenen Dentalrestauration endbehandelt wird,
oder bei dem
(d2) einem Rohling mit Schichten von Zirkonoxid gemäß Anspruch 12 durch maschinelle Bearbeitung die Form der Dentalrestauration gegeben wird, wobei die maschinelle Bearbeitung vorzugsweise mit computergesteuerten Fräs- und/oder Schleifvorrichtungen erfolgt,
(e2) mindestens eine Wärmebehandlung durchgeführt wird, um das ungesinterte oder vorgesinterte Zirkonoxid in dichtgesintertes Zirkonoxid umzuwandeln, und
(f2) gegebenenfalls die Oberfläche der erhaltenen Dentalrestauration endbehandelt wird.

19. Verfahren nach Anspruch 17 oder 18, bei dem die Dentalrestauration ausgewählt ist aus der Gruppe von Kronen, Abutments, Abutmentkronen, Inlays, Onlays, Veneers, Schalen, Brücken und Überwürfen.

20. Verwendung eines Rohlings nach einem der Ansprüche 1 bis 13 zur Herstellung von Dentalrestaurationen und insbesondere von Kronen, Abutments, Abutmentkronen, Inlays, Onlays, Veneers, Schalen, Brücken und Überwürfen.

## Claims

1. Blank (1) for dental purposes which comprises a first and a second layer (3, 5) which, independently of each other, are based on
glass,
glass-ceramic or
ceramic,
wherein the first layer and the second layer differ in colour and form a boundary surface (7), wherein the boundary surface runs obliquely, wherein the blank has a rotation axis and an insertion axis, wherein the rotation axis is the axis about which the blank is rotated during the machining for shaping the desired dental restoration, and the insertion axis is the axis running in the cervical-incisal direction,
wherein in a first sectional plane (15) through the blank, which runs parallel to the insertion axis (8) of the blank, the boundary surface (7) has an angle of 10 to 70°, preferably 10 to 60°, to the rotation axis of the blank,
wherein the angle between the insertion axis (8) and the rotation axis (9) of the blank in the first sectional plane is from 70 to 110°.

2. Blank according to claim 1, in which in a first sectional plane through the blank, which runs parallel to the insertion axis of the blank, the boundary surface does not run perpendicular to the insertion axis.

3. Blank according to claim 1 or 2, in which the boundary surface in the first sectional plane runs substantially straight, wherein the boundary surface in the first sectional plane is preferably at an angle of from 20 to 80°, particularly preferably of from 30 to 80° to the insertion axis
or
in which the boundary surface in the first sectional plane runs arcuately and the line of best fit through the arcuate boundary surface in the first sectional plane is preferably at an angle of from 20 to 80°, particularly preferably 30 to 80° to the insertion axis.

4. Blank according to any one of claims 1 to 3, in which the angle between the insertion axis and the rotation axis of the blank in the first sectional plane is from 80 to 100° and particularly preferably about 90°.

5. Blank according to any one of claims 1 to 4, in which the boundary surface (7) runs arcuately through the blank in a second sectional plane (23) which runs perpendicular to the first sectional plane, wherein the boundary surface preferably runs convexly curved in the second sectional plane.

6. Blank according to claim 5, in which the boundary surface in the second sectional plane has a mamelon structure (25), wherein the mamelon structure preferably has indentations, the depth of which is up to 2 mm, preferably from 0.1 to 0.5 mm.

7. Blank according to any one of the preceding claims, in which the first layer has a refractive index which differs from the refractive index of the second layer by not more than 0.1
and/or
which comprises a mark which is recognized by a CAD/CAM device and with which the position of the boundary surface can be determined with an accuracy of in particular 0.1 mm.

8. Blank according to any one of the preceding claims, in which the glass, the glass-ceramic or the ceramic are selected from lithium silicate glass, lithium silicate glass-ceramic, silicon dioxide glass, silicon dioxide glass-ceramic and/or zirconium oxide.

9. Blank according to claim 8, in which the first and second layer, independently of each other, are based on
lithium silicate glass,
lithium silicate glass with nuclei or
lithium metasilicate glass-ceramic,
wherein the lithium silicate glass, the lithium silicate glass with nuclei or the lithium metasilicate glass-ceramic comprise at least one and preferably all of the following components in the amounts indicated:
| Component | wt.-% |
|---|---|
| SiO₂ | 64.0 to 73.0 |
| Li₂O | 12.0 to 18.0 |
| K₂O | 1.0 to 5.0 |
| Al₂O₃ | 0.5 to 5.0 |
| P₂O₅ | 1.0 to 7.0, |
wherein the amount of oxides of elements with an atomic number of 19 or higher in the first layer differs preferably by not more than 2 wt.-% from the amount of oxides of elements with an atomic number of 19 or higher in the second layer.

10. Blank according to claim 8, in which the first and second layer, independently of each other, are based on
silicon dioxide glass,
silicon dioxide glass with nuclei or
silicon dioxide glass-ceramic,
wherein the silicon dioxide glass, the silicon dioxide glass with nuclei or the silicon dioxide glass-ceramic comprise at least one and preferably all of the following components in the amounts indicated:
| Component | wt.-% |
|---|---|
| SiO₂ | 58.0 to 92.0 |
| Li₂O | 2.0 to 10.0 |
| Me^{I}₂O | 0 to 13.0 |
| Me^{II}O | 0 to 11.0 |
| Me^{III}₂O₃ | 0 to 10.0 |
| Me^{IV}O₂ | 0 to 21.0 |
| P₂O₅ | 0 to 7.0 |
| Me^{V}₂O₅ | 0 to 6.0 |
| Me^{VI}O₃ | 0 to 6.0 |
| Fluorine | 0 to 5.0, |
wherein Me^{I}₂O is selected in particular from Na₂O, K₂O, Rb₂O and/or Cs₂O; Me^{II}O is selected in particular from MgO, CaO, SrO and/or ZnO; Me^{III}₂O₃ is selected in particular from Al₂O₃, B₂O₃, Y₂O₃, La₂O₃, Ga₂O₃ and/or In₂O₃; Me^{IV}O₂ is selected in particular from ZrO₂, GeO₂, CeO₂, TiO₂ and/or SnO₂; Me^{V}₂O₅ is selected in particular from V₂O₅, Ta₂O₅ and/or Nb₂O₂; and Me^{VI}O₃ is selected in particular from WO₃ and/or MoO₃.

11. Blank according to any one of claims 9 or 10, in which the first and second layer are monolithic.

12. Blank according to claim 8, in which the first and second layer, independently of each other, comprise
unsintered zirconium oxide or
presintered zirconium oxide.

13. Blank according to any one of the preceding claims, which comprises a holder (29) for a processing device, wherein the holder consists in particular of metal or plastic.

14. Process for the production of a blank according to any one of claims 1 to 13, in which
(a) a first layer based on glass, glass-ceramic or ceramic is provided,
(b) the surface of the first layer is shaped in order to provide the desired course of the boundary surface of the first and second layer of the blank, and
(c) a second layer based on glass, glass-ceramic or ceramic is applied to the surface of the first layer.

15. Process for the production of a blank with layers of lithium silicate glass, lithium silicate glass with nuclei, lithium metasilicate glass-ceramic, silicon dioxide glass, silicon dioxide glass with nuclei or silicon dioxide glass-ceramic according to any one of claims 9 to 11, in which
(a1) a first layer of lithium silicate glass, lithium silicate glass with nuclei, lithium metasilicate glass-ceramic, silicon dioxide glass, silicon dioxide glass with nuclei or silicon dioxide glass-ceramic with a viscosity of at least 6.6 Pa·s is provided in a mould,
(b1) the surface of the first layer is shaped in order to provide the desired course of the boundary surface of the first and second layer of the blank, and
(c1) a second layer of lithium silicate glass, lithium silicate glass with nuclei, lithium metasilicate glass-ceramic, silicon dioxide glass, silicon dioxide glass with nuclei or silicon dioxide glass-ceramic is applied to the surface of the first layer.

16. Process for the production of a blank with layers of zirconium oxide according to claim 12, in which
(a2) a first layer of unsintered or dispersed zirconium oxide is provided in a mould,
(b2) the surface of the first layer is shaped in order to provide the desired course of the boundary surface of the first and second layer of the blank, and
(c2) a second layer of unsintered or dispersed zirconium oxide is applied to the surface of the first layer.

17. Process for the production of a dental restoration, in which
(d) a blank according to any one of claims 1 to 13 is given the shape of the dental restoration by machining, wherein the machining is effected preferably with computer-controlled milling and/or grinding devices,
(e) optionally at least one heat treatment is carried out, and
(f) optionally the surface of the dental restoration obtained is finished.

18. Process according to claim 17, in which
(d1) a blank according to claim 9 or 11 is given the shape of the dental restoration by machining, wherein the machining is effected preferably with computer-controlled milling and/or grinding devices,
(e1) at least one heat treatment is carried out in order to convert the lithium silicate glass, the lithium silicate glass with nuclei or the lithium metasilicate glass-ceramic into lithium disilicate glass-ceramic, and
(f1) optionally the surface of the dental restoration obtained is finished,
or in which
(d2) a blank with layers of zirconium oxide according to claim 12 is given the shape of the dental restoration by machining, wherein the machining is effected preferably with computer-controlled milling and/or grinding devices,
(e2) at least one heat treatment is carried out in order to convert the unsintered or presintered zirconium oxide into densely sintered zirconium oxide, and
(f2) optionally the surface of the dental restoration obtained is finished.

19. Process according to claim 17 or 18, in which the dental restoration is selected from the group of crowns, abutments, abutment crowns, inlays, onlays, veneers, facets, bridges and overstructures.

20. Use of a blank according to any one of claims 1 to 13 for the production of dental restorations and in particular of crowns, abutments, abutment crowns, inlays, onlays, veneers, facets, bridges and overstructures.

## Revendications

1. Ébauche (1) destinée à des fins dentaires, présentant une première et une deuxième couche (3, 5) qui sont réalisées indépendamment l'une de l'autre à base de verre, de vitrocéramique ou de céramique, la première couche et la deuxième couche se distinguant par la couleur et formant une interface (7), l'interface s'étendant en biais, l'ébauche présentant un axe de rotation et un axe d'insertion, l'axe de rotation désignant l'axe autour duquel l'ébauche est tournée lors de l'usinage mécanique en vue de la mise en forme de la restauration dentaire souhaitée, et l'axe d'insertion désignant l'axe qui s'étend dans la direction cervicale-incisale,
sachant que dans un premier plan de coupe (15) à travers l'ébauche, qui s'étend parallèlement à l'axe d'insertion (8) de l'ébauche, l'interface (7) présente un angle allant de 10 à 70°, de préférence de 10 à 60°, par rapport à l'axe de rotation de l'ébauche,
sachant que l'angle entre l'axe d'insertion (8) et l'axe de rotation (9) de l'ébauche est de 70 à 110° dans le premier plan de coupe.

2. Ébauche selon la revendication 1, dans laquelle, dans un premier plan de coupe à travers l'ébauche, qui s'étend parallèlement à l'axe d'insertion de l'ébauche, l'interface ne s'étend pas perpendiculairement à l'axe d'insertion.

3. Ébauche selon la revendication 1 ou 2, dans laquelle l'interface s'étend de façon sensiblement rectiligne dans le premier plan de coupe, l'interface présentant, dans le premier plan de coupe, de préférence un angle allant de 20 à 80°, et de manière particulièrement avantageuse de 30 à 80°, par rapport à l'axe d'insertion,
ou
dans laquelle l'interface s'étend sous forme d'arc dans le premier plan de coupe, et la droite de compensation passant par l'interface en forme d'arc présente, dans le premier plan de coupe, de préférence un angle allant de 20 à 80°, et de manière particulièrement avantageuse de 30 à 80°, par rapport à l'axe d'insertion.

4. Ébauche selon une des revendications 1 à 3, dans laquelle l'angle entre l'axe d'insertion et l'axe de rotation de l'ébauche est compris entre 80 et 100° dans le premier plan de coupe, et est de manière particulièrement avantageuse de 90°.

5. Ébauche selon une des revendications 1 à 4, dans laquelle l'interface (7) s'étend en forme d'arc dans un deuxième plan de coupe (23) à travers l'ébauche, qui s'étend perpendiculairement au premier plan de coupe, l'interface s'étendant de préférence sous une forme bombée convexe dans le deuxième plan de coupe.

6. Ébauche selon la revendication 5, dans laquelle l'interface présente une structure à mamelons (25) dans le deuxième plan de coupe, la structure de mamelon comportant de préférence des encoches dont la profondeur va jusqu'à 2 mm, et est de préférence comprise entre 0,1 et 0,5 mm.

7. Ébauche selon une des revendications précédentes, dans laquelle la première couche présente un indice de réfraction qui ne diffère pas de plus de 0,1 de l'indice de réfraction de la deuxième couche,
et/ou
qui présente un repère pouvant être reconnu par un dispositif CAO/FAO et permettant de déterminer la position de l'interface avec une précision de notamment 0,1 mm.

8. Ébauche selon une des revendications précédentes, dans laquelle le verre, la vitrocéramique ou la céramique sont choisis parmi le verre au silicate de lithium, la vitrocéramique au silicate de lithium, le verre au dioxyde de silicium, la vitrocéramique au dioxyde de silicium et/ou la zircone.

9. Ébauche selon la revendication 8, dans laquelle la première et la deuxième couche sont réalisées indépendamment l'une de l'autre à base de
verre au silicate de lithium,
verre au silicate de lithium comportant des germes, ou
vitrocéramique au métasilicate de lithium,
où le verre au silicate de lithium, le verre au silicate de lithium comportant des germes ou la vitrocéramique au métasilicate de lithium contiennent au moins un et de préférence l'ensemble des constituants suivants, dans les quantités indiquées :
| Constituant | % en poids |
|---|---|
| SiO₂ | 64,0 à 73,0 |
| Li₂O | 12,0 à 18,0 |
| K₂O | 1,0 à 5,0 |
| Al₂O₃ | 0,5 à 5,0 |
| P₂O₅ | 1,0 à 7,0, |
où la quantité d'oxydes d'éléments ayant un numéro atomique de 19 ou plus, dans la première couche, ne diffère de préférence pas de plus de 2 % en poids de la quantité d'oxydes d'éléments ayant un numéro atomique de 19 ou plus, dans la deuxième couche.

10. Ébauche selon la revendication 8, dans laquelle la première et la deuxième couche sont réalisées indépendamment l'une de l'autre à base de
verre au dioxyde de silicium,
verre au dioxyde de silicium comportant des germes,
vitrocéramique au dioxyde de silicium,
où le verre au dioxyde de silicium, le verre au dioxyde de silicium comportant des germes ou la vitrocéramique au dioxyde de silicium contiennent au moins un et de préférence l'ensemble des constituants suivants, dans les quantités indiquées :
| Constituant | % en poids |
|---|---|
| SiO₂ | 58,0 à 92,0 |
| Li₂O | 2,0 à 10,0 |
| Me^{I}₂O | 0 à 13,0 |
| Me^{II}O | 0 à 11,0 |
| Me^{III}₂O₃ | 0 à 10,0 |
| Me^{IV}O₂ | 0 à 21,0 |
| P₂O₅ | 0 à 7,0, |
| Me^{V}₂O₅ | 0 à 6,0 |
| Me^{VI}O₃ | 0 à 6,0 |
| Fluor | 0 à 5,0, |
où Me^{I}₂O est choisi notamment parmi Na₂O, K₂O, Rb₂O et/ou Cs₂O ; Me^{II}O est choisi notamment parmi MgO, CaO, SrO et/ou ZnO ; Me^{III}₂O₃ est choisi notamment parmi Al₂O₃, B₂O₃, Y₂O₃, La₂O₃, Ga₂O₃ et/ou In₂O₃ ; Me^{IV}O₂ est choisi notamment parmi ZrO₂, GeO₂, CeO₂, TiO₂ et/ou SnO₂; Me^{V}₂O₅ est choisi notamment parmi V₂O₅, Ta₂O₅ et/ou Nb₂O₅ ; et Me^{VI}O₃ est choisi notamment parmi WO₃ et/ou MoO₃.

11. Ébauche selon la revendication 9 ou 10, dans laquelle la première et la deuxième couche sont monolithiques.

12. Ébauche selon la revendication 8, dans laquelle la première et la deuxième couche contiennent indépendamment l'une de l'autre de la
zircone non frittée ou de la
zircone préfrittée.

13. Ébauche selon une des revendications précédentes, qui présente un support (29) pour un dispositif d'usinage, le support étant constitué notamment de métal ou de matière plastique.

14. Procédé de fabrication d'une ébauche selon une des revendications 1 à 13, selon lequel
(a) une première couche à base de verre, vitrocéramique ou céramique est mise en place,
(b) la surface de la première couche est mise en forme pour fournir le tracé souhaité de l'interface de la première et de la deuxième couche de l'ébauche, et
(c) une deuxième couche à base de verre, vitrocéramique ou céramique est appliquée sur la surface de la première couche.

15. Procédé de fabrication d'une ébauche avec des couches de verre au silicate de lithium, verre au silicate de lithium comportant des germes, vitrocéramique au métasilicate de lithium, verre au dioxyde de silicium, verre au dioxyde de silicium comportant des germes ou vitrocéramique au dioxyde de silicium, selon une des revendications 9 à 11, selon lequel
(a1) une première couche de verre au silicate de lithium, verre au silicate de lithium comportant des germes, vitrocéramique au métasilicate de lithium, verre au dioxyde de silicium, verre au dioxyde de silicium comportant des germes ou vitrocéramique au dioxyde de silicium, ayant une viscosité d'au moins 6,6 Pa·s, est mise en place dans un moule,
(b1) la surface de la première couche est mise en forme pour fournir le tracé souhaité de l'interface de la première et de la deuxième couche de l'ébauche, et
(c1) une deuxième couche de verre au silicate de lithium, verre au silicate de lithium comportant des germes, vitrocéramique au métasilicate de lithium, verre au dioxyde de silicium, verre au dioxyde de silicium comportant des germes ou vitrocéramique au dioxyde de silicium est appliquée sur la surface de la première couche.

16. Procédé de fabrication d'une ébauche avec des couches de zircone, selon la revendication 12, selon lequel
(a2) une première couche de zircone non frittée ou dispersée est mise en place dans un moule,
(b2) la surface de la première couche est mise en forme pour fournir le tracé souhaité de l'interface de la première et de la deuxième couche de l'ébauche, et
(c2) une deuxième couche de zircone non frittée ou dispersée est appliquée sur la surface de la première couche.

17. Procédé de réalisation d'une restauration dentaire, selon lequel
(d) une ébauche selon une des revendications 1 à 13 reçoit par usinage mécanique la forme de la restauration dentaire, l'usinage mécanique s'effectuant de préférence avec des dispositifs de fraisage et/ou de polissage commandés par ordinateur,
(e) le cas échéant au moins un traitement thermique est effectué, et
(f) le cas échéant, la surface de la restauration dentaire obtenue fait l'objet d'un traitement final.

18. Procédé selon la revendication 17, selon lequel
(d1) une ébauche selon la revendication 9 ou 11 reçoit par usinage mécanique la forme de la restauration dentaire, l'usinage mécanique s'effectuant de préférence avec des dispositifs de fraisage et/ou de polissage commandés par ordinateur,
(e1) au moins un traitement thermique est effectué pour transformer le verre au silicate de lithium, le verre au silicate de lithium comportant des germes ou la vitrocéramique au métasilicate de lithium en vitrocéramique au disilicate de lithium, et
(f1) le cas échéant, la surface de la restauration dentaire obtenue fait l'objet d'un traitement final,
ou selon lequel
(d2) une ébauche comportant des couches de zircone selon la revendication 12 reçoit par usinage mécanique la forme de la restauration dentaire, l'usinage mécanique s'effectuant de préférence avec des dispositifs de fraisage et/ou de polissage commandés par ordinateur,
(e2) au moins un traitement thermique est effectué pour transformer la zircone non frittée ou préfrittée en zircone frittée à densité maximale, et
(f2) le cas échéant, la surface de la restauration dentaire obtenue fait l'objet d'un traitement final.

19. Procédé selon la revendication 17 ou 18, selon lequel la restauration dentaire est choisie dans le groupe des couronnes, piliers, couronnes à piliers, inlays, onlays, facettes, coquilles, bridges et éléments de recouvrement.

20. Utilisation d'une ébauche selon une des revendications 1 à 13, pour la fabrication de restaurations dentaires et notamment de couronnes, piliers, couronnes à piliers, inlays, onlays, facettes, coquilles, bridges et éléments de recouvrement.
